# EUROPÄISCHE PATENTSCHRIFT

(11) **EP 0 675 203 B1**
(45) Veröffentlichungstag und Bekanntmachung des Hinweises auf die Patenterteilung: **12.12.2001**
(21) Anmeldenummer: 95102032.0
(22) Anmeldetag: 14.02.1995
(51) Int. Cl.: C12N 15/63

(54) **Integrationsvektoren zur Herstellung von Genen, die rekombinante Antikörper codieren**
Integrationvectors for producing genes, which produce recombinant antibodies
Vecteurs d'intégration pour la préparation de gênes pour la production d'anticorps recombinants

(30) Priorität: 28.02.1994 DE 4406512; 01.06.1994 DE 4419254
(43) Veröffentlichungstag der Anmeldung: 04.10.1995
(73) Patentinhaber: GSF-Forschungszentrum für Umwelt und Gesundheit GmbH, 85764 Oberschleissheim (DE)
(72) Erfinder: Mocikat, Ralph, Dr., D-81639 München (DE)
(74) Vertreter: VOSSIUS & PARTNER

(56) Entgegenhaltungen:
- EUROPEAN JOURNAL OF IMMUNOLOGY, Bd. 25, 1995 Seiten 792-797, C. KARDINAL ET AL. 'Integration vectors for antibody chimerization by homologous recombination in hybridoma cells'
- MOL. GEN. GENET., Bd. 242, 1994 Seiten 528-538, P. LANG ET AL. 'Replacement-like recombination induced by an integration vector with a murine homology flank at the immunoglobulin heavy-chain locus in mouse and rat hybridoma cells'

## Beschreibung

Die Erfindung betrifft Integrationsvektoren zur Herstellung von Genen, die rekombinante Antikörper codieren, Verfahren zur Herstellung von rekombinanten Antikörpern unter Verwendung dieser Vektoren, Verfahren zur Herstellung von Zellinien unter Verwendung dieser Vektoren, die nur die rekombinanten Antikörper produzieren, sowie Kits zur Herstellung von rekombinanten Antikörpern, die mindestens einen dieser Vektoren enthalten.

Homologe Rekombination zwischen exogenen und chromosomalen DNA-Sequenzen hat Anwendungen in vielen Bereichen der modernen Biologie erfahren. So ist über homologe Rekombinationen das Genom embryonaler Stammzellen und weiterer Säugerzelltypen spezifisch verändert worden (vgl. z.B. Capecchi, Science 244 (1989), 1288-1292). Durch diese Veränderungen wurde beispielsweise die Genfunktion zerstört, es wurden Mutationen korrigiert oder geringfügige Änderungen in der Nukleotidsequenz eines Gens vorgenommen (vgl. z.B. Davis et al., Mol. Cell. Biol. 12 (1992), 2769-2776). Man weiß, daß die exogenen Nukleinsäuren in Hefe sequenzspezifisch eingebaut werden (Orr-Weaver und Szostak, Microbiol. Rev. 49 (1985), 33-58). In Säugerzellen erfolgt der Einbau hingegen mit geringer Häufigkeit über homologe Rekombination zugunsten einer wesentlich häufigeren zufälligen Integration.

Ausgehend von dieser Erkenntnis ist versucht worden, wirksame Selektions- und Absuchverfahren zu entwickeln, welche die Isolierung von Säugerzellen mit einem gewünschten Rekombinationsereignis ermöglichen. Mansour beispielsweise hat dazu ein positiv-negatives Selektionsverfahren vorgeschlagen (Mansour et al., Nature 336 (1988), 348-352). Man weiß mittlerweile, daß die Effizienz der homologen Rekombination von der Länge der homologen Sequenzen abhängt (vgl. z.B. Berinstein et al., Mol. Cell. Biol. 12 (1992), 360-367), wie auch durch die Transkriptionsaktivität der Zielsequenz beeinflußt wird (vgl. z.B. Nicholoff, Mol. Cell. Biol. 12 (1992), 5311-5318).

Für homologe Rekombinationsexperimente stehen zwei im Prinzip unterschiedliche Vektoren zur Verfügung, die als "Integrations-" und "Replacementvektoren" bezeichnet werden. Integrationsvektoren enthalten eine homologe flankierende Sequenz, in die ein Bruch des DNA-Doppelstranges eingefügt wird. Bei dem Rekombinationsereignis wird der gesamte Vektor in das Genom integriert, was zu einer Duplikation eines Teils der Zielsequenz führt. Replacementvektoren dagegen enthalten eine homologe Sequenz, in die ein heterologer Bereich eingefügt ist (beispielsweise ein Gen für einen Selektionsmarker). Diese Vektorart wird vor der Transfektion am Ende oder außerhalb der homologen Region linearisiert. Das Integrationsmuster von Integrationsvektoren in Hefe- und Säugerzellen kann durch das von Orr-Weaver und Szostak entwickelte Doppelstrangbruch-Reparaturmodell hinreichend vorhergesagt werden (Orr-Weaver und Szostak, a.a.O., Szostak et al., Cell. 33 (1983), 25-35). Replacementvektoren mit nichthomologen Enden scheinen hingegen einen anderen Integrationsmechanismus zu verwenden, da ihre freien Strangenden eine weniger wichtige Rolle für die Integrationsart zu spielen scheinen als die von Integrationsvektoren (Hasty et al., Mol. Cell. Biol. 12 (1992), 2464-2474).

Von besonderer Wichtigkeit für eine hohe Anzahl homologer Rekombinationsereignisse ist die Verwendung isogener DNA (vgl. z.B. Deng und Capecchi, Mol. Cell. Biol. 12 (1992), 3365-3371), da Basenfalschpaarungen innerhalb der homologen Region die Rekombinationshäufigkeit drastisch herabsetzen. Sofern isogene homologe Sequenzen verwendet werden, sind Integrations- und Replacementvektoren ähnlich wirksam. Werden allerdings nicht-isogene Sequenzen benutzt, so werden mit Replacementvektoren weniger gewünschte Rekombinationsereignisse erzielt. Dies ist darauf zurückzuführen, daß die Integration von Replacementvektoren zwei Rekombinationsereignisse erfordert. Bei Basenfehlpaarungen kommt es dadurch weniger häufig zu einer erfolgreichen homologen Rekombination als bei Integrationsvektoren, die nur ein Rekombinationsereignis benötigen (Deng und Capecchi, a.a.O.).

Die homologe Rekombinationstechnik hat man sich auch für die Konstruktion chimärer Antikörper zunutze gemacht. Eine Vielzahl von monoclonalen Maus-Antikörpern mit therapeutisch interessanten Spezifitäten sind nämlich für die Therapie im Menschen wertlos, da ihre Applikation die Induktion von gegen sie gerichteten Antikörper hervorruft. Dies führt zu einer Neutralisierung der Antikörper und damit des therapeutischen Effektes. Daneben können unerwünschte Nebenwirkungen hervorrufen werden. Fell et al., Proc. Natl. Acad. Sci. USA 86 (1989), 8507-8511 haben die variable Region der schweren Kette eines Maus-Antikörpers über homologe Rekombination unter Verwendung eines Integrationsvektors mit der menschlichen C_{γ1}-Region verknüpft. Sie haben dabei ein Konstrukt verwendet; das die Maus J_{H}4-Region, einen Teil des C_{µ}-Introns mit dem C_{µ}-Enhancer, verbunden mit den menschlichen C_{γ1}-Exons sowie einen Selektionsmarker enthielt. Mit diesem Konstrukt haben Fell et al. eine Häufigkeit von einer funktionellen genomischen Rekombination in 200 Transfektanten erhalten. Eine derartige Häufigkeit an homologen Rekombinationsereignissen macht ein relativ aufwendiges Absuchverfahren nach den gewünschten Klonen noch immer erforderlich.

Andererseits sind menschliche Zellinien verfügbar, die Antikörper bestimmter Spezifität sezernieren. Auch hier wäre wünschenswert, wenn diese Antikörper auf einfache Weise mit anderen Spezifitäten, die beispielsweise aus der Maus stammen oder synthetischen Ursprungs sein können, versehen werden könnten. Bislang sind solche rekombinanten Antikörper im wesentlichen durch die rekombinante DNA-Technologie in nicht-produzierenden Zellinien mit sehr geringer Ausbeute hergestellt worden. Dies ist ein aufwendiges Verfahren, das zum raschen Austausch von Antikörperspezifitäten ungeeignet ist.

Der vorliegenden Erfindung lag daher das technische Problem zugrunde, Vektoren bereitzustellen, mit denen sich eine große Häufigkeit homologer Rekombinationen in Immunglobulinloci erzielen läßt, wodurch mit größerer Häufigkeit als mit den im Stand der Technik bekannten Vektoren funktionelle rekombinante Antikörper bereitgestellt werden können. Die Lösung dieses technischen Problems wird durch die in den Ansprüchen dargestellten Ausführungsformen erzielt.

Somit betrifft die Erfindung einen Integrationsvektor zur Herstellung von Genen, die rekombinante Antikörper codieren, der die folgenden Elemente enthält:
(a) einen Bereich von mindestens 1,5 kb, der homolog zu einem Bereich des µ-Introns oder k-Introns ist, der keinen oder keinen funktionellen C_{µ}- oder Cₖ-Enhancer enthält;
(b) mindestens eine DNA-Sequenz, die eine Domäne eines Antikörpers oder einen Teil davon codiert; und
(c) einen in eukaryontischen Antikörper-produzierenden Zellen selektierbaren Marker, der keinen funktionellen Enhancer-Bereich aufweist, wobei die Expression dieses Markers nach der Integration vom zellulären C_{µ}- oder Cₖ-Enhancer gesteuert wird.

Der erfindungsgemäße Vektor kann einen zu einem Intron-Bereich homologen Bereich von mindestens 1,5 kb aufweisen, in dem der Ig-Enhancer natürlicherweise nicht vorkommt oder aus dem er deletiert wurde oder in dem er inaktiviert wurde.

Die DNA-Sequenz kann beispielsweise ein oder mehrere Exons umfassen, solange eine funktionelle Domäne eines Antikörpers oder ein funktioneller Teil davon codiert wird. Ist der funktionelle Teil der Domäne Teil einer V-Domäne, so muß diese zur Bindung an das Zielantigen fähig sein oder dazu beitragen. Handelt es sich um einen Teil einer C-Domäne, so muß diese mindestens einen Teil der Effektorfunktionen ausüben können.

In einer bevorzugten Ausführungsform umfaßt der Bereich des µ- oder k-Introns von mindestens 1,5 kb den Bereich, in dem der C_{µ}- oder Cₖ-Enhancer lokalisiert ist, wobei dieser Bereich keinen funktionellen C_{µ}- oder Cₖ-Enhancer mehr enthält.

Der Enhancer kann in dieser Ausführungsform entweder deletiert oder inaktiviert sein. Eine solche Inaktivierung kann z.B. durch Mutagenese nach im Stand der Technik bekannten Verfahren herbeigeführt werden.

Bei dem in eukaryontischen Antikörper-produzierenden Zellen selektierbaren Marker ist der Enhancer vorzugsweise deletiert oder inaktiviert worden. In einer anderen Ausführungsform weist der Marker keinen natürlichen Enhancer auf.

Mit dem erfindungsgemäßen Integrationsvektor lassen sich vorteilhafterweise verschiedene V-Gene oder verschiedene Isotypen in ein funktionell rearrangiertes Antikörpergen einrekombinieren. Der Integrationsvektor muß im Gegensatz zu einem entsprechenden Replacementvektor lediglich eine zur genomischen Sequenz homologe Sequenz aufweisen. Damit muß keine der jeweiligen endogenen konstanten Region homologe Sequenz, wie bei den im Stand der Technik bekannten Replacementvektoren, in den Vektor eingebaut werden. Sofern darüber hinaus das verwendete Hybridom nicht-isogen zu der Vektorsequenz ist, läßt sich mit dem Integrationsvektor eine höhere Ausbeute erzielen als mit einem Replacementvektor, d.h. ein und derselbe Integrationsvektor läßt sich auf Hybridome unterschiedlicher Mausstämme anwenden.

Die im Vektor enthaltene homologe Sequenz muß eine Länge von mindestens 1,5 kb aufweisen, um ein homologes Rekombinationsereignis überhaupt erzielen zu können. Diese DNA-Sequenz von mindestens 1,5 kb kann aus verschiedenen Bereichen des C_{µ}- oder Cₖ-Introns gewählt werden. Der Enhancer selbst ist erfindungsgemäß nicht im Konstrukt enthalten oder aus der Homologieflanke deletiert bzw. darin inaktiviert worden. Bei der Integration des Vektors in die homologe Sequenz eines funktionell rearrangierten Antikörpergens wird die Expression des rekombinanten Gens unter die Kontrolle des endogenen Enhancers gestellt. Werden Exons, die konstante Regionen codieren, einrekombiniert, so stehen diese ferner unter der Kontrolle des endogenen V-Promotors. Der Enhancer reguliert darüber hinaus die Expression des selektierbaren Markers. Dadurch wird sichergestellt, daß der Selektionsmarker nur dann aktiv wird, wenn er in der Nähe eines starken Enhancers integriert wird. Durch die verwendete Homologieflanke wird eine homologe Rekombination mit dem Immunglobulinlocus begünstigt, wodurch der selektierbare Marker unter die Kontrolle des endogenen C_{µ}- oder Cₖ-Enhancers gestellt wird.

Der Begriff "rekombinanter Antikörper", wie hier verwendet, bezeichnet jeden nicht natürlicherweise vorkommenden Antikörper. Dabei können die im Integrationsvektor eingefügten DNA-Sequenzen natürlichen, synthetischen oder semi-synthetischen Ursprungs sein. Wie natürliche Antikörper codierende Sequenzen kloniert werden bzw. wie synthetische oder semi-synthetische DNA-Sequenzen hergestellt werden, ist dem Fachmann aus dem Stand der Technik bekannt; vgl. z.B. Sambrook et al., "Molecular Cloning, A Laboratory Manual", 2. Auflage 1989, Cold Spring Harbor Laboratory, Cold Spring Harbor, USA, sowie Harlow und Lane "Antibodies, A Laboratory Manual" 1988, Cold Spring Harbor Laboratory, Cold Spring Harbor, USA.

In einer bevorzugten Ausführungsform des erfindungsgemäßen Integrationsvektors codiert die DNA-Sequenz eine V-Domäne oder einen Teil davon. Vorzugsweise ist die die V-Domäne codierende Sequenz ein Vₖ-Gen, wenn das Integrationsereignis in einem endogenen k-Gen stattfinden soll. Sofern das Rekombinationsereignis in einem schwere Kette-Gen erfolgen soll, ist die die V-Domäne codierende Sequenz vorzugsweise ein V_{H}-Gen. Die V-Region kann jede mögliche Antigenspezifität aufweisen. In dieser Ausführungsform ist der Selektionsmarker 5' von der die V-Domäne codierenden Sequenz angeordnet, während der Intron-Bereich 3' davon positioniert ist. Codiert die DNA-Sequenz nur einen Teil einer V-Domäne, so muß diese zur Antigenbindung geeignet sein.

In einer anderen bevorzugten Ausführungsform weist der Integrationsvektor eine DNA-Sequenz auf, die eine konstante Region oder einen Teil davon codiert. Diese Sequenz kann entweder für die schwere oder die leichte Kette eines Antikörpers codieren. Dem Fachmann ist bekannt, daß bei allen Isotypen mehrere Exons für die schwere Kette codieren. Sofern nur ein C_{H}-Exon für die schwere Kette im Konstrukt vorhanden ist, ist dies vorzugsweise das C_{H1}-Exon. Mit einem derartigen Konstrukt können rekombinante Antikörper hergestellt werden, die die Funktionalität von Fab- oder F(ab)₂-Fragmenten aufweisen. Vorzugsweise enthält der erfindungsgemäße Integrationsvektor jedoch sämtliche Exons eines schwere Kette-Isotyps, wodurch eine vollständige schwere Kette exprimiert werden kann. In dieser Ausführungsform sind die Elemente (a), (b) und (c) in dieser Reihenfolge aufeinanderfolgend in 5'→3' Richtung angeordnet.

In einer anderen bevorzugten Ausführungsform des erfindungsgemäßen Integrationsvektors weist der zum µ- oder k-Intron homologe Bereich eine Länge von mindestens 1,9 kb auf.

In einer weiteren bevorzugten Ausführungsform des erfindungsgemäßen Integrationsvektors weist dieser zum µ- oder k-Intron homologe Bereich eine Länge von mindestens 2,0 kb auf.

In einer weiteren bevorzugten Ausführungsform enthält der erfindungsgemäße Integrationsvektor eine Bakterien-kompatible Regulationseinheit. Eine derartige Bakterien-kompatible Regulationseinheit ermöglicht die Klonierung und Amplifizierung des Vektors in bakteriellen Systemen, beispielsweise in E. coli. Bakterien-kompatible Regulationssysteme sind dem Fachmann aus dem Stand der Technik bekannt; vgl. Sambrook et al., a.a.O. Ein Beispiel für eine derartige Bakterien-kompatible Regulationseinheit ist die Regulationseinheit aus dem Plasmid pBR322.

In einer anderen bevorzugten Ausführungsform dient der Integrationsvektor zur Herstellung eines rekombinanten Antikörpers, der ein chimärer Antikörper ist. Unter "chimärer Antikörper" wird hier ein Antikörper verstanden, der die V- und C-Regionen aus verschiedenen Spezies kombiniert. Beispielsweise kann ein V-Gen aus der Maus mit den C-Exons eines menschlichen Isotyps kombiniert werden.

In einer anderen bevorzugten Ausführungsform des erfindungsgemäßen Vektors codiert die DNA-Sequenz Domänen einer menschlichen Antikörperkette. Diese Domänen können sowohl V-als auch C-Domänen oder Teile davon sein.

In einer weiteren bevorzugten Ausführungsform des erfindungsgemäßen Vektors codiert die DNA-Sequenz Domänen, die aus der Maus, Ratte, Ziege, aus dem Pferd oder Schaf stammen. Vorzugsweise stammen sämtliche DNA-Sequenzen für entweder die V- oder die C-Regionen aus einer dieser Tierarten. Die Erfindung umfaßt jedoch auch solche Ausführungsformen, in denen die die C-Regionen codierenden Sequenzen aus verschiedenen Tierarten entnommen wurden.

Wie auch die in einer anderen Ausführungsform des erfindungsgemäßen Vektors verwendeten menschliche Domänen codierenden DNA-Sequenzen können diese Domänen codierende DNA-Sequenzen für die homologe Rekombination mit der entsprechenden Sequenz aus einer anderen Säugerart eingesetzt werden.

In einer anderen bevorzugten Ausführungsform des erfindungsgemäßen Vektors codiert die DNA-Sequenz sämtliche C-Domänen eines sekretorischen Antikörpers.

In einer weiteren bevorzugten Ausführungsform des erfindungsgemäßen Integrationsvektors codiert die DNA-Sequenz sämtliche C-Domänen eines membrangebundenen Antikörpers.

Eine weitere bevorzugte Ausführungsform des erfindungsgemäßen Vektors enthält eine DNA-Sequenz, die C-Domänen eines Antikörpers codiert, der ein IgM-, IgG1-, IgG2a-, IgG2b-, IgG3-, IgG4-, IgA-, IgD- oder IgE-Antikörper ist. Dem Fachmann ist bekannt, daß manche dieser Isotypen nicht in allen Säugerspezies vorkommen. So enthält das menschliche Genom beispielsweise C-Gene, die IgG4-Isotypen, nicht aber IgG2a-oder IgG2b-Isotypen codieren. Dagegen enthält das Maus-Genom C-Gene, die den IgG2a- und den IgG2b-Isotyp, nicht aber den IgG4-Isotyp codieren.

In einer weiteren Ausführungsform des erfindungsgemäßen Vektors sind die rekombinanten Antikörper-produzierenden Zellen menschliche Zellen oder Mauszellen oder Hybride davon. Mauszellen werden dann bevorzugt, wenn der Integrationsvektor ein menschliches C-Gen/menschliche C-Gene enthält, das/die stromabwärts von der V-Region eines rearrangierten Maus-Antikörpergens einrekombiniert werden soll(en). Der so exprimierte Antikörper kann beispielsweise für diagnostische und vorzugsweise für therapeutische Verfahren verwendet werden. Weiterhin werden für therapeutische Verfahren einzusetzende Antikörper vorzugsweise in menschlichen Zellen hergestellt.

So können z.B. V-Gene aus der Maus mit gewünschten Spezifitäten in ein funktionelles menschliches Immunglobulin-Gen, vorzugsweise ein IgGl- oder IgG3-Gen einrekombiniert werden. Ferner hat die Verwendung menschlicher Zellen den Vorteil, daß die Antikörper mit einem menschlichen Glykosilierungsmuster versehen werden, das bei therapeutischer Applikation keine unerwünschten Nebenwirkungen hervorruft.

Dem Fachmann ist bekannt, wie er Hybride aus menschlichen Zellen und Mauszellen herstellen kann. Solche Hybride lassen sich beispielsweise durch Fusion von Maus-B-Lymphoblasten mit menschlichen Myelomzellen herstellen.

In all diese Zellen können mit dem erfindungsgemäßen Integrationsvektor ferner synthetische V-Gene einrekombiniert werden. Ist die endogene C-Region eine menschliche C-Region, so lassen sich damit humanisierte Antikörper herstellen.

In einer weiteren bevorzugten Ausführungsform des erfindungsgemäßen Integrationsvektors ist der selektierbare Marker gpt, neo oder codiert für Hygromycin-Resistenz. Der Fachmann ist mit der Züchtung von Zellen unter Selektionsbedingungen, die diese Marker erfordern, vertraut (vgl. z.B. Sambrook et al., a.a.O.). Er ist darüber hinaus in der Lage, weitere Selektionsmarker auszuwählen, die im erfindungsgemäßen Vektor verwendet werden können.

In einer besonders bevorzugten Ausführungsform trägt der erfindungsgemäße Integrationsvektor die Bezeichnung pSV232Agpt-hu_{γ1}-X5. Seine Konstruktion ist in Beispiel 1 beschrieben.

Die Erfindung betrifft ferner ein Verfahren zur Herstellung von rekombinanten Antikörpern, bei denen man die folgenden Schritte durchführt
(a) Transfektion von Antikörper-produzierenden Zellen mit dem erfindungsgemäßen Vektor;
(b) Selektion von stabilen Transformanten; und
(c) Identifizierung der den gewünschten Antikörper produzierenden Zellen.

Die Transfektion Antikörper-produzierender Zellen gilt als Standardverfahren der modernen Immunologie. Dem Fachmann ist bekannt, daß die Transfektionsbedingungen für jede verwendete Zellinie eingestellt werden müssen. Ein Leitfaden zur Errichtung solcher Transfektionsbedingungen ist beispielsweise in Toneguzzo et al., Mol. Cell Biol. 6 (1986), 703-706 sowie in der Beschreibung zum Biorad "Genepulser" gegeben. Für die Mausmyelomlinie NS-1 (ATCC TIB 18) beispielsweise sind geeignete Transfektionsbedingungen in Mocikat et al., Gene 136 (1993), 349-353 beschrieben. Die Selektion von stabilen Transformanten erfolgt durch Züchtung der Transformanten für mindestens 7 Tage in einem geeigneten Selektionsmedium. Die Selektion stabiler Transformanten ist notwendig, um Zellen abzutöten, die das Plasmid nicht aufgenommen haben. Die Wahl des Selektionsmediums richtet sich selbstverständlich nach dem verwendeten Selektionsmarker. Die Herstellung geeigneter Selektionsmedien ist im Stand der Technik bekannt und beispielsweise in Sambrook et al., a.a.O. nachlesbar.

Die Identifizierung der den gewünschten Antikörper-produzierenden Zellen kann beispielsweise durch für die konstante Region spezifische Antikörper erfolgen, wenn die DNA-Sequenz im Integrationsvektor ein C-Gen oder eine Domäne davon codiert. Wird hingegen ein V-Gen durch den Integrationsvektor in ein funktionell rearrangiertes endogenes Antikörpergen einrekombiniert, so kann die Expression des gewünschten Antikörpers durch anti-idiotypische Antikörper bestimmt werden. Als Testverfahren eignen sich beispielsweise RIAs oder ELISAs. Auch diese Verfahren gehören zum Standardrepertoire des Durchschnittsfachmanns. Darüber hinaus kann die Integration der transferierten Domäne(n) mit dem im Stand der Technik bekannten PCR-Verfahren ermittelt werden. Der Fachmann ist in der Lage, hierzu geeignete Primer und Reaktionsbedingungen zu bestimmen.

In einer bevorzugten Ausführungsform des erfindungsgemäßen Verfahrens erfolgt die Transfektion durch Elektroporation, Calcium-Kopräzipitation, Lipofektion, die DEAE-Dextran-Technik oder retroviralen Gentransfer. All diese Verfahren sind im Stand der Technik gut bekannt. Der Fachmann weiß daher, wie er die Bedingungen für die einzelnen Transfektionsverfahren im erfindungsgemäßen Verfahren einzustellen hat.

In einer anderen bevorzugten Ausführungsform des erfindungsgemäßen Verfahrens erfolgt die Selektion in einem Medium, das als Selektionsmittel Mykophenolsäure, G418, oder Hygromycin enthält. Wie bereits vorstehend erwähnt, sind diese Selektionsmittel im Stand der Technik gut bekannt. Ihre Wahl hängt vom verwendeten Selektionsmarker ab, während ihre Dosierung aus Standardwerken der Molekularbiologie hergeleitet werden kann; vgl. z.B. Sambrook et al., a.a.O.

In einer anderen bevorzugten Ausführungsform des erfindungsgemäßen Verfahrens codiert die DNA-Sequenz konstante Domänen des γ₁-, γ₂ₐ-, γ_{2b}-, γ₃-, γ₄-, µ-, α-, δ- oder ε-Isotyps.

In einer weiteren bevorzugten Ausführungsform des erfindungsgemäßen Verfahrens wird zusätzlich folgender Schritt durchgeführt:
- (da): Retransfektion der Antikörper-produzierenden Zellen mit dem erfindungsgemäßen Integrationsvektor, der einen anderen Selektionsmarker als den in Schritt (a) verwendeten aufweist; oder
- (db): Retransfektion der Antikörper-produzierenden Zellen mit einem Integrationsvektor, der mindestens einen funktionellen Enhancer enthält und der einen anderen Selektionsmarker als den in Schritt (a) verwendeten aufweist.

Der funktionelle Enhancer ist vorzugsweise der c_{µ}-, c_{κ}- oder SV40-Enhancer.

Die durch homologe Rekombination erzeugten Zellinien exprimieren oft zusätzlich zu den gewünschten rekombinanten Antikörpern weiterhin den ursprünglichen endogenen Antikörper. Dies ist auf das Vorhandensein mehrerer Kopien funktionell rearrangierter Allele in der Antikörper produzierenden Zelle, beispielsweise einer Hybridomzelle zurückzuführen. Mit dem erfindungsgemäßen Verfahren wird eine derartige unerwünschte Doppelproduktion von Antikörpern ausgeschaltet. Eine nach der Selektion von Schritt (b) erhaltene stabile Transformante, die nach homologer Rekombination zwei Antikörper-Spezies sezerniert, wird erfindungsgemäß mit demselben Integrationsvektor, der jedoch mit einem anderen Selektionsmarkergen versehen ist, als zur ersten Transfektion von Schritt (a) verwendet wurde oder mit einem entsprechenden Enhancer enthaltenden Integrationsvektor, retransfiziert. Ein derartiger Austausch von Genen für Selektionsmarker kann nach im Stand der Technik bekannten einfachen DNA-Rekombinationstechniken erreicht werden. Mögliche austauschbare Selektionsmarkergene wurden vorstehend in dieser Beschreibung erwähnt. Nach Selektion auf den zweiten Selektionsmarker werden Clone etabliert, die nur noch den gewünschten rekombinanten Antikörper exprimieren. Durch diese zweite Transfektion und anschließende Selektion wird darüber hinaus eine weitere Steigerung der Antikörper-Produktionsrate erreicht.

Schließlich betrifft die Erfindung Kits zur Herstellung von rekombinanten Antikörpern, die mindestens einen erfindungsgemäßen Vektor enthalten. Diese Kits können zur leichten Herstellung von Antikörpern mit gewünschten Spezifitäten oder gewünschten Isotypen verwendet werden. Sie bieten dem Fachmann ein Mittel, mit relativ wenig Aufwand gewünschte rekombinante Antikörper zu isolieren. Die erfindungsgemäßen Kits enthalten entweder einen oder mehrere erfindungsgemäße Vektoren. Sie können darüber hinaus weitere Komponenten, wie beispielsweise geeignete Selektionsmittel enthalten.

Die Figuren zeigen:

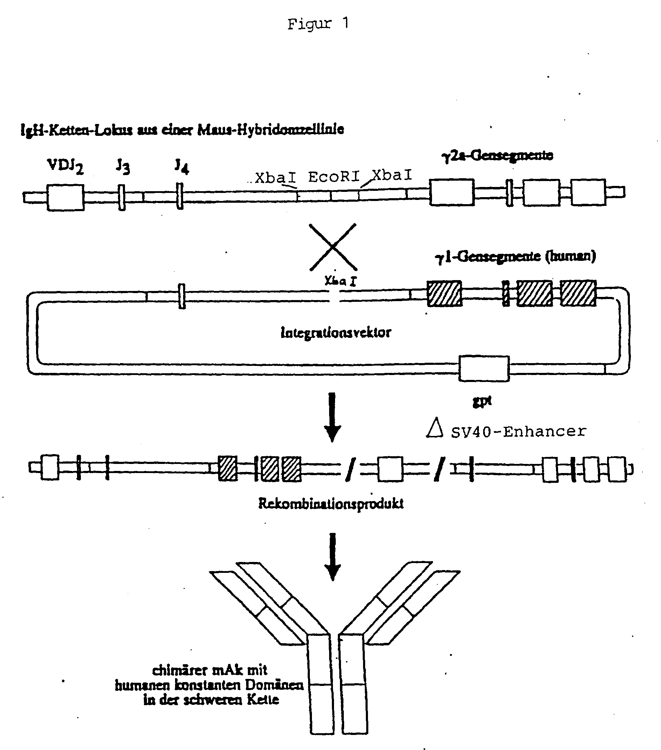

Figur 1: schematische Darstellung einer Ausführungsform des erfindungsgemäßen Integrationsvektors, mit dem in ein funktionell rearrangiertes Maus γ₂ₐ-Gen die C-Exons für das menschliche γ₁-Gen eingefügt werden. Der Vektor wird vor der Transfektion in eine geeignete Maus-Hybridomzellinie mit dem Restriktionsenzym XbaI linearisiert. Nach der Transfektion integriert der Vektor an der durch X gekennzeichneten Stelle. Durch die Integration werden Teile des Maus-Genoms dupliziert. Das so erhaltene Rekombinationsprodukt ist ebenfalls schematisch dargestellt.

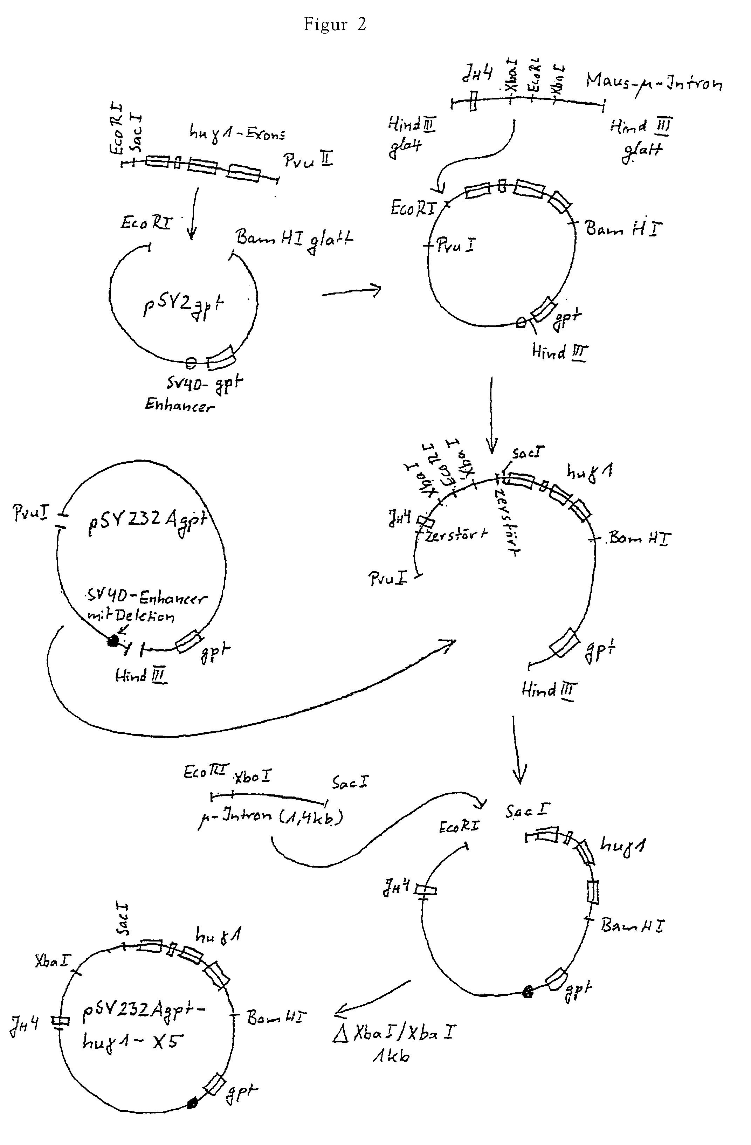

Figur 2: Schematische Darstellung der Konstruktion des Vektors pSV232Agpt-huγ1-X5. Die einzelnen Konstruktionsschritte sind genauer in Beispiel 1 beschrieben.

Die Beispiele erläutern die Erfindung.

### Beispiel 1: Vektorkonstruktion

Die humanen konstanten IgG1-Gensegmente werden als 2,9 kb-EcoRI-PvuII-Fragment (Ellison et al., Nucleic Acids Res. 10 (1982), 4071-4079) in den mit EcoRI und BamHI gespaltenen Vektor pSVgpt (Mulligan und Berg, Proc. Natl. Acad. Sci. USA 78 (1981), 2072-2076) kloniert, dessen BamHI-Stelle durch Behandlung mit Klenow-Enzym (2 Einheiten, 30 Min. bei 37°C) mit glatten Enden versehen worden ist. In die EcoRI-Stelle stromaufwärts von den C-Exons wird anschließend über glatte Enden (nach Klenow-Behandlung, 2 Einheiten, 30 Min. bei 37°C) ein 2,3 kb-HindIII-Fragment eingefügt, das einen Teil des Maus-µ-Introns einschließlich des µ-Enhancers sowie das J_{H}4-Segment enthält (Gough und Bernard, Proc. Natl. Acad. Sci. USA 78 (1981), 509-513; Banerji et al., Cell 33 (1983), 729-740). Aus diesem Konstrukt wird mit PvuI und HindIII ein Bereich herausgeschnitten, der Teile des pBR-Vektors, die murine Flanke, die humanen IgG1-C-Exons sowie Teile der gpt-Expressionseinheit ohne den SV40-Enhancer enthält. Dieses Fragment wird mit einem 3,7 kb großen Bereich aus dem Plasmid pSV232Agpt (Kadesch und Berg, Mol. Cell. Biol. 6 (1986), 2593-2601) ligiert, das durch Spaltung mit PvuI und HindIII aus diesem Vektor isoliert worden ist. Dieses Fragment enthält den SV40-Promotor, sowie einen nicht mehr funktionellen Teil des SV40-Enhancers. Schließlich wird die 0,7 kb lange Sequenz stromabwärts von der EcoRI-Stelle durch Spaltung mit EcoRI und SacI aus dem Vektor eliminiert und durch ein 1,4 kb-Fragment aus dem murinen µ-Intron ersetzt, so daß die gesamte Homologieflanke eine Länge von 3,0 kb aufweist. Aus dieser Flanke wird durch Spaltung mit XbaI, wodurch ein Fragment von 1,0 kb entsteht, und Rezirkularisierung der Bereich des µ-Intron-Enhancers entfernt. Der resultierende Vektor wird mit pSV232Agpt-huγ₁-X5 bezeichnet; vgl. Figur 2.

### Beispiel 2: Transfektionsexperiment

Zur Transfektion werden 10⁷ exponentiell wachsende Hybridomzellen abzentrifugiert und in 700 µl eiskaltem RPMI 1640 (Gibco, BRL) resuspendiert. 20 µg pSV232Agpt-huγ₁-X5 werden mit XbaI vollständig linearisiert, mit Isopropanol präzipitiert, in 20 µl H₂O bidest. resuspendiert und zu der Zellsuspension gegeben. Die Transfektion erfolgt mittels Elektroporation (Biorad, Typ "Genepulser") mit einem einzelnen Stromimpuls; Feldstärke und Kondensatorkapazität werden für jede Zellinie individuell eingestellt. Der Fachmann ist mit der Einstellung dieser Bedingungen vertraut, die beispielsweise in der Betriebsableitung zum "Genepulser" oder in Toneguzzo et al., a.a.O. detailliert wiedergegeben sind. Die Zellen werden 10 Minuten auf Eis gehalten und dann in einer Dichte von 10⁵ Zellen pro Kavität auf 24-Loch-Platten in RPMI 1640, supplementiert mit 10% foetalem Kälberserum (Gibco, BRL) und 2mM Glutamin (Gibco, BRL) verteilt.

### Beispiel 3: Selektion stabiler Transfektanten

48 Stunden nach der Transfektion beginnt die Selektion mit steigenden Konzentrationen von Mykophenolsäure bis zu einer Endkonzentration von 2 µg/ml in Gegenwart von 250 µg/ml Xanthin und 15 µg/ml Hypoxanthin. Die Klone werden auf stabiles Wachstum hin im Abstand von 2 Tagen abgesucht. Die Überstände stabil wachsender Klone werden auf ihren Gehalt an humanem IgG1 abgesucht. Dazu wird ein ELISA mit Ziege-anti-human-IgGFc-spezifischem Fänger-Antikörper und Peroxidase-gekoppeltem Detektions-Antikörper derselben Spezifität verwendet (vgl. Beispiel 4). Klone, deren Überstand ein positives Ergebnis liefert, werden expandiert.

### Beispiel 4: Enzymgebundener Absorptionstest

ELISA-Platten (Nunc) werden mit Ziege-anti-human-IgGFc-spezifischem Fänger-Antikörper (Dianova, Hamburg) beschichtet. Freie Bindungsstellen werden mit 1% Milchpulver blockiert. Anschließend werden die zu testenden Kulturüberstände inkubiert und der Test anschließend mit Peroxydase-gekoppeltem Detektions-Antikörper (Dianova) inkubiert. Nach jedem der vorstehenden Schritte werden die Platten mit Phosphat-gepufferter physiologischer Kochsalzlösung ausgiebig gewaschen. Der Test wird mit o-Phenylendiamin (Sigma) entwickelt. Jede der vorstehend genannten Reaktionen wird eine Stunde bei Raumtemperatur durchgeführt. Die Farbreaktion wird in einem ELISA-Meßgerät (STLLabinstruments, Typ SF+) bei einer Wellenlänge von 405nm gemessen.

Nach der Transfektion von 10⁷ Hybridomzellen werden 20 Mykophenol-resistente Klone erhalten. Nach statistischer Auswertung exprimieren davon 5% den menschlichen γ₁-Isotyp. Die Integrität der menschlichen schweren Kette wurde im SDS-PAGE-Gel und im Western-Blot (vgl. Beispiel 5) bestätigt.

Die Antigen-Bindungsspezifität und die Affinität entsprechen der des parentalen Maus-Antikörpers, wie durch Kompetitionsversuche (vgl. Beispiel 6) nachgewiesen wurde.

### Beispiel 5: Western Blot

Die rekombinanten Antikörper werden aus dem Kulturüberstand über eine FPLC-Protein-G-Sepharose-Säule (Pharmacia, Freiburg) gereinigt. Mit 2µg Protein wird eine SDS-PAGE (Gradient von 8-15% Acrylamid) nach Standardverfahren durchgeführt. Die Proteine werden durch Elektroblotten auf eine Nitrocellulosemembran übertragen und durch Ziege-anti-human-IgGFc-Antikörper, der an Meerrettichperoxidase gekoppelt ist (Dianova) nachgewiesen. Die Farbentwicklung wird durch Zugabe von 3,3-Diaminobenzidin eingeleitet.

### Beispiel 6: Kompetitionsexperimente

Maus-Lymphozytenzellen, die das Zielantigen tragen, werden 30 Min. nach Standardverfahren mit Verdünnungsreihen des rekombinanten Antikörpers und anschließend 10 Min. mit dem parentalen Antikörper, der mit FITC markiert ist, bei 0°C inkubiert. Die Beladung der Zellen mit dem markierten Antikörper wird nach Standardverfahren im FACS gemessen.

### Beispiel 7: Ausschaltung des ursprünglichen endogenen Nager-Antikörpers in einem durch homologe Rekombination entstandenen Doppelproduzenten

Ein 4,4 kb großes Fragment, das aus dem Plasmid pSVneo (Southern und Berg, J. Mol. Appl. Genet. 1 (1982) 327-341) nach Spaltung des Plasmids mit PvuI und BamHI isoliert wird und das das neo-Gen trägt, wird in den Integrationsvektor eingebaut, der den funktionellen SV40- und c_{µ}-Enhancer enthält und aus dem mit derselben Spaltung das gpt-Gen entfernt wurde. In diesen Vektor wird der SV40-Enhancer durch den trunkierten SV40-Enhancer aus pSV232Agpt ersetzt und außerdem der µ-Intron-Enhancer deletiert, wie in Beispiel 1 bzw. Figur 2 beschrieben. Eine Zellinie, die sowohl einen humanen als auch den parentalen Maus-Isotyp sezerniert, wird entweder mit dem Enhancer-tragenden oder Enhancer-losen, neo-tragenden Vektor transfiziert, wie in Beispiel 2 beschrieben. 48 Stunden später beginnt die Selektion mit 1,5 mg/ml G418 (Gibco BRL), wobei die Selektion mit Mykophenolsäure beibehalten wird (vgl. Beispiel 3). Die Überstände stabiler Transfektanten werden in einem ELISA abgesucht, wie in Beispiel 4 beschrieben, wobei jedoch als Fänger- und als Detektions-Antikörper Ziege-anti-Maus-IgGFc verwendet wird. Die Clone, die den murinen Antikörper nicht mehr exprimieren, werden expandiert.
In der Regel wird die Antikörper-Produktionsrate mit dem in diesem Beispiel beschriebenen Verfahren um mindestens einen Faktor 2 im Vergleich zu den in den vorherigen Beispielen beschriebenen Verfahren verbessert.

## Patentansprüche

1. Integrationsvektor zur Herstellung von Genen, die rekombinante Antikörper codieren, der die folgenden Elemente enthält:
(a) einen Bereich von mindestens 1,5 kb, der homolog zu einem Bereich des µ-Introns oder k-Introns ist, der keinen oder keinen funktionellen C_{µ}- oder Cₖ-Enhancer enthält;
(b) mindestens eine DNA-Sequenz, die eine Domäne eines Antikörpers oder einen Teil davon codiert; und
(c) einen in eukaryontischen Antikörper-produzierenden Zellen selektierbaren Marker, der keinen funktionellen Enhancer-Bereich aufweist, wobei die Expression dieses Markers nach der Integration vom zellulären C_{µ}- oder Cₖ-Enhancer gesteuert wird.

2. Vektor nach Anspruch 1, wobei der Bereich des µ- oder k-Introns den C_{µ}- oder Cₖ-Enhancerlokus umfaßt und der Bereich von mindestens 1,5 kb keinen funktionellen C_{µ}- oder Cₖ-Enhancer enthält.

3. Vektor nach Anspruch 1 oder 2, wobei die DNA-Sequenz eine V-Domäne oder einen Teil davon codiert.

4. Vektor nach Anspruch 1 oder 2, wobei die DNA-Sequenz eine konstante Region oder einen Teil davon codiert.

5. Vektor nach einem der Ansprüche 1 bis 4, wobei der Bereich, der homolog zu einem den C_{µ}- oder Cₖ-Enhancer umfassenden Bereich des µ- oder k-Introns ist, mindestens 1,9 kb umfaßt.

6. Vektor nach einem der Ansprüche 1 bis 4, wobei der Bereich, der homolog zu einem den C_{µ}- oder Cₖ-Enhancer umfassenden Bereich des µ- oder k-Introns ist, mindestens 2,0 kb umfaßt.

7. Vektor nach einem der Ansprüche 1 bis 6, der eine Bakterien-kompatible Regulationseinheit enthält.

8. Vektor nach einem der Ansprüche 1 bis 7, wobei der rekombinante Antikörper ein chimärer Antikörper ist.

9. Vektor nach einem der Ansprüche 1 bis 8, wobei die DNA-Sequenz Domänen einer menschlichen Antikörperkette codiert.

10. Vektor nach einem der Ansprüche 1 bis 8, wobei die DNA-Sequenz Domänen codiert, die aus der Maus, Ratte, Ziege, aus dem Pferd oder Schaf stammen.

11. Vektor nach einem der Ansprüche 1, 2 und 4 bis 10, wobei die DNA-Sequenz sämtliche C-Domänen eines sekretorischen Antikörpers codiert.

12. Vektor nach einem der Ansprüche 1, 2 und 4 bis 10, wobei die DNA-Sequenz sämtliche C-Domänen eines membrangebundenen Antikörpers codiert.

13. Vektor nach einem der Ansprüche 1, 2 und 4 bis 12, wobei die Exons C-Domänen eines Antikörpers codieren, der ein IgM-, IgG1-, IgG2a-, IgG2b-, IgG3-, IgG4-, IgA-, IgD- oder IgE-Antikörper ist.

14. Vektor nach einem der Ansprüche 1 bis 13, wobei die rekombinante Antikörper produzierenden Zellen menschliche Zellen oder Mauszellen oder Hybride davon sind.

15. Vektor nach einem der Ansprüche 1 bis 14, wobei der selektierbare Marker gpt, neo, oder ein Hygromycin-Resistenz codierender Marker ist.

16. Vektor nach Anspruch 15, der die Bezeichnung pSV232Agpt-huγ₁-X5 trägt und die in Figur 2 beschriebenen Merkmale aufweist.

17. Verfahren zur Herstellung von rekombinanten Antikörpern, bei dem man die folgenden Schritte durchführt:
(a) Transfektion von Antikörper-produzierenden Zellen mit einem Vektor nach einem der Ansprüche 1 bis 16;
(b) Selektion von stabilen Transformanten; und
(c) Identifizierung der den gewünschten Antikörper produzierenden Zellen.

18. Verfahren nach Anspruch 17, wobei die Transfektion durch Elektroporation, Calciumphosphat-Kopräzipitation, Lipofektion, die DEAE-Dextran-Technik, oder retroviralen Gentransfer erfolgt.

19. Verfahren nach Anspruch 17 oder 18, wobei die Selektion in einem Medium erfolgt, das als Selektionsmittel Mykophenolsäure, G418 oder Hygromycin enthält.

20. Verfahren nach einem der Ansprüche 17 bis 19, wobei die Exons konstante Domänen des µ-, γ₁-, γ₂ₐ-, γ_{2b}-, γ₃-, γ₄-, δ-, α- oder ε-Isotyps codieren.

21. Verfahren nach einem der Ansprüche 17 bis 20, bei dem man zusätzlich folgenden Schritt durchführt:
(da) Retransfektion der Antikörper-produzierenden Zellen mit dem Vektor nach einem der Ansprüche 1 bis 16, der einen anderen Selektionsmarker als den in Schritt (a) verwendeten aufweist; oder
(db) Retransfektion der Antikörper-produzierenden Zellen mit einem Integrationsvektor, der mindestens einen funktionellen Enhancer enthält und der einen anderen Selektionsmarker als den in Schritt (a) verwendeten aufweist.

22. Kit zur Herstellung von rekombinanten Antikörpern, enthaltend mindestens einen Vektor nach einem der Ansprüche 1 bis 16.

## Claims

1. An integration vector for producing genes encoding recombinant antibodies containing the following elements:
(a) a region of at least 1.5 kb which is homologous to a region of the µ-intron or the k-intron which contains no or no functional C_{µ} or Cₖ enhancer;
(b) at least one DNA sequence encoding a domain of an antibody or a part thereof; and
(c) a marker which is selectable in eukaryotic cells producing antibodies and which does not have a functional enhancer region, wherein the expression of said marker is controlled by the cellular C_{µ} or Cₖ enhancer after the integration.

2. The vector according to claim 1, wherein the region of the µ- or k-intron comprises the C_{µ} or Cₖ enhancer locus and wherein the region of at least 1.5 kb does not have any functional C_{µ} or Cₖ enhancer.

3. The vector according to claim 1 or 2, wherein the DNA sequence encodes a V-domain or a part thereof.

4. The vector according to claim 1 or 2, wherein the DNA sequence encodes a constant region or a part thereof.

5. The vector according to any one of claims 1 to 4, wherein the region which is homologous to the region of the µ- or k-intron comprising the C_{µ} or Cₖ enhancer comprises at least 1.9 kb.

6. The vector according to any one of claims 1 to 4, wherein the region which is homologous to the region of the p- or k-intron comprising the C_{µ} or Cₖ enhancer comprises at least 2.0 kb.

7. The vector according to any one of claims 1 to 6 containing a regulatory unit which is compatible with bacteria.

8. The vector according to any one of claims 1 to 7, wherein the recombinant antibody is a chimeric antibody.

9. The vector according to any one of claims 1 to 8, wherein the DNA sequence encodes domains of a human antibody chain.

10. The vector according to any one of claims 1 to 8, wherein the DNA sequence encodes domains which are derived from mouse, rat, goat, horse or sheep.

11. The vector according to any one of claims 1, 2 and 4 to 10, wherein the DNA sequence encodes all C-domains of a secretory antibody.

12. The vector of any one of claims 1, 2 and 4 to 10, wherein the DNA sequence encodes all C-domains of a membrane-bound antibody.

13. The vector according to any one of claims 1, 2 and 4 to 12, wherein the exons encode the C-domains of an antibody which is an IgM, IgG1, IgG2a, IgG2b, IgG3, IgG4, IgA, IgD or IgE antibody.

14. The vector according to any one of claims 1 to 13, wherein the cells producing the recombinant antibodies are human cells or mouse cells or hybrids thereof.

15. The vector according to any one of claims 1 to 14, wherein the selectable marker is a gpt marker, a neo marker or a marker encoding a resistance against hygromycin.

16. The vector according to claim 15 which is designated pSV232Agpt-huγ₁-X5 and has the features described in Figure 2.

17. A method for producing recombinant antibodies, wherein the following steps are carried out:
(a) transfecting antibody-producing cells with a vector according to any one of claims 1 to 16;
(b) selecting stable transformants; and
(c) identifying the cells which produce the desired antibody.

18. The method according to claim 17, wherein the transfection is carried out by electroporation, calcium phosphate co-precipitation, lipofection, DEAE dextrane technique or retroviral gene transfer.

19. The method according to claim 17 or 18, wherein the selection is carried out in a medium which contains mycophenolic acid, G418 or hygromycin as a selection agent.

20. The method according to any one of claims 17 to 19, wherein the exons encode constant domains of the µ-, γ₁-, γ₂ₐ-, γ_{2b}-, γ₃-, γ₄-, δ-, α- or ε-isotype.

21. The method according to any one of claims 17 to 20, wherein, in addition, the following steps are carried out:
(da) retransfecting the antibody-producing cells with the vector according to any one of claims 1 to 16 which contains a selection marker other than the one used in step (a); or
(db) retransfecting the antibody-producing cells with an integration vector which contains at least one functional enhancer and a selection marker other than the one used in step (a).

22. A kit for producing recombinant antibodies which contains at least one vector according to any one of claims 1 to 16.

## Revendications

1. Vecteur d'intégration pour la production de gènes codant pour des anticorps recombinants, qui contient les éléments suivants :
(a) une région d'au moins 1,5 kb qui est homologue d'une région de l'intron µ ou de l'intron k qui ne contient aucun activateur C_{µ} ou Cₖ ou aucun activateur C_{µ} ou Cₖ fonctionnel ;
(b) au moins une séquence d'ADN qui code pour un domaine d'un anticorps ou une partie de celui-ci ; et
(c) un marqueur sélectionnable dans des cellules eucaryotes productrices d'anticorps, qui ne comporte aucune région d'activateur fonctionnel, l'expression de ce marqueur étant régulée par l'activateur cellulaire C_{µ} ou Cₖ après l'intégration.

2. Vecteur selon la revendication 1, dans lequel la région de l'intron µ ou k comprend le locus d'activateur C_{µ} ou Cₖet la région d'au moins 1,5 kb ne contient aucun activateur C_{µ} ou Cₖ fonctionnel.

3. Vecteur selon la revendication 1 ou 2, dans lequel la séquence d'ADN code pour un domaine V ou une partie de celui-ci.

4. Vecteur selon la revendication 1 ou 2, dans lequel la séquence d'ADN code pour une région constante ou une partie de celui-ci.

5. Vecteur selon l'une quelconque des revendications 1 à 4, dans lequel la région qui est homologue d'une région de l'intron µ ou k, comprenant l'activateur C_{µ} ou Cₖ, comprend au moins 1,9 kb.

6. Vecteur selon l'une quelconque des revendications 1 à 4, dans lequel la région qui est homologue d'une région de l'intron µ ou k, comprenant l'activateur C_{µ} ou Cₖ, comprend au moins 2,0 kb.

7. Vecteur selon l'une quelconque des revendications 1 à 6, qui contient une unité de régulation compatible avec les bactéries.

8. Vecteur selon l'une quelconque des revendications 1 à 7, dans lequel l'anticorps résultant est un anticorps chimère.

9. Vecteur selon l'une quelconque des revendications 1 à 8, dans lequel la séquence d'ADN code pour des domaines d'une chaîne d'anticorps humain.

10. Vecteur selon l'une quelconque des revendications 1 à 8, dans lequel la séquence d'ADN code pour des domaines qui sont issus de la souris, du rat, de la chèvre, du cheval ou du mouton.

11. Vecteur selon l'une quelconque des revendications 1, 2 et 4 à 10, dans lequel la séquence d'ADN code pour l'ensemble des domaines C d'un anticorps sécrétoire.

12. Vecteur selon l'une quelconque des revendications 1, 2 et 4 à 10, dans lequel la séquence d'ADN code pour l'ensemble des domaines C d'un anticorps fixé à la membrane.

13. Vecteur selon l'une quelconque des revendications 1 à 13, dans lequel les exons des domaines C codent pour un anticorps qui est un anticorps IgM, IgG1, IgG2a, IgG2b, IgG3, IgG4, IgA, IgD ou IgE.

14. Vecteur selon l'une quelconque des revendications 1 à 13, dans lequel les cellules produisant des anticorps recombinants sont des cellules humaines ou des cellules murines ou des hybrides de celles-ci.

15. Vecteur selon l'une quelconque des revendications 1 à 14, dans lequel le marqueur sélectionnable est gpt, neo ou un marqueur codant pour la résistance à l'hygromycine.

16. Vecteur selon la revendication 15, qui porte la désignation psV232Agpt -huγ₁-X5 et présente les caractéristiques décrites sur la figure 2.

17. Procédé pour la production d'anticorps recombinants, dans lequel on effectue les étapes suivantes :
(a) transfection de cellules produisant des anticorps avec un vecteur selon l'une quelconque des revendications 1 à 16 ;
(b) sélection de transformants stables ; et
(c) identification des cellules produisant les anticorps recherchés.

18. Procédé selon la revendication 17, dans lequel la transfection s'effectue par électroporation, précipitation au phosphate de calcium, lipofection, la technique au DEAE-dextrane ou le transfert de gènes rétroviraux.

19. Procédé selon la revendication 17 ou 18, dans lequel on effectue la sélection dans un milieu qui contient comme agent de sélection l'acide mycophénolique, G418 ou l'hygromycine.

20. Procédé selon l'une quelconque des revendications 17 à 19, dans lequel les exons codent pour des domaines constants de l'isotype µ, γ₁, γ₂ₐ, γ_{2b}, γ₃, γ₄, δ, α ou ε.

21. Procédé selon l'une quelconque des revendications 17 à 20, dans lequel on effectue en outre l'étape suivante :
(da) retransfection des cellules produisant des anticorps avec le vecteur selon l'une quelconque des revendications 1 à 16, qui comporte un autre marqueur de sélection que celui utilisé dans l'étape (a) ; ou
(db) retransfection des cellules produisant des anticorps avec un vecteur d'intégration qui contient au moins un activateur fonctionnel et comporte un autre marqueur de sélection que celui utilisé dans l'étape (a).

22. Nécessaire pour la production d'anticorps recombinants, contenant au moins un vecteur selon l'une quelconque des revendications 1 à 16.
